# FASCICULE DE BREVET EUROPEEN

(11) **EP 0 975 597 B1**
(45) Date de publication et mention de la délivrance du brevet: **02.09.2009**
(21) Numéro de dépôt: 98920581.0
(22) Date de dépôt: 07.04.1998
(51) Int. Cl.: C07D 215/14

(54) **DERIVES DE QUINOLEINES EN TANT QU'INHIBITEURS DE LA VIH INTEGRASE**
CHINOLINDERIVATE MIT INSBESONDERE ANTIVIRALEN EIGENSCHAFTEN, HERSTELLUNG UND BIOLOGISCHE ANWENDUNGEN DAVON
QUINOLINE DERIVATIVES, HAVING IN PARTICULAR ANTIVIRAL PROPERTIES, PREPARATION AND BIOLOGICAL APPLICATIONS THEREOF

(30) Priorité: 08.04.1997 FR 9704289
(43) Date de publication de la demande: 02.02.2000
(73) Titulaire: CENTRE NATIONAL DE LA RECHERCHE SCIENTIFIQUE (CNRS), 75794 Paris Cedex 16 (FR); Institut Gustave Roussy, 94805 Villejuif Cédex (FR)
(72) Inventeur: MEKOUAR, Khalid, F-75010 Paris (FR); D'ANGELO, Jean, F-91300 Massy (FR); DESMAELE, Didier, F-94260 Fresnes (FR); MOUSCADET, Jean-François, F-75012 Paris (FR); SUBRA, Frédéric, F-75018 Paris (FR); AUCLAIR, Christian, F-75011 Paris (FR)
(74) Mandataire: Habasque, Etienne J. Jean-François
(86) Numéro de dépôt international: PCT/FR1998/000701
(87) Numéro de publication internationale: WO 1998/045269

(56) Documents cités:
- EP-A- 0 206 751
- EP-A- 0 219 307
- EP-A- 0 256 180
- EP-A- 0 286 089
- EP-A- 0 318 093
- EP-A- 0 643 045
- EP-A- 0 725 063
- WO-A-94/27968
- WO-A-96/02506
- WO-A-96/04246
- DE-A- 3 405 395
- CHEMICAL ABSTRACTS, vol. 77, no. 7, 14 août 1972 Columbus, Ohio, US; abstract no. 47548q, TAK,B.K. ET AL: "Influence of solvent interaction on the absorption spectra of some phenol betaines. II." XP002051760 & J. INDIAN CHEM. SOC., vol. 49, no. 2, - 1972 pages 139-144,
- BURKE T R ET AL: "HYDROXYLATED AROMATIC INHIBITORS OF HIV-1 INTEGRASE" JOURNAL OF MEDICINAL CHEMISTRY, vol. 38, no. 21, 13 octobre 1995, pages 4171-4178, XP000569362
- J.P.PHILLIPS ET AL.: "Styryl derivatives of 8-quinolinol" JOURNAL OF ORGANIC CHEMISTRY, vol. 24, - 1959 pages 1104-1106, XP002077558

## Description

La présente invention a pour objet des dérivés de quinoléines dotés notamment de propriétés inhibitrices de l'intégrase du virus de l'immunodéficience humaine.

Elle vise également un procédé de synthèse de ces dérivés et leurs applications biologiques.

L'intégration de l'ADN génomique du VIH dans les chromosomes de la cellule infectée est strictement nécessaire à la réplication du virus. L'enzyme virale qui catalyse l'intégration de l'ADN viral dans la chromatine de l'hôte est l'intégrase. En conséquence, un inhibiteur de l'intégrase constitue *ipso facto* un candidat pour bloquer l'infection par le VIH, et éventuellement un agent thérapeutique efficace. Des trois enzymes virales qui conditionnent la réplication du VIH, à savoir la réverse transcriptase, la protéase et l'intégrase, l'intégrase est la dernière à n'être la cible d'aucun agent thérapeutique. Dans le contexte de la polythérapie qui semble être à l'heure actuelle l'unique voie à même de combattre efficacement l'évolution rapide du virus, l'obtention d'un inhibiteur de l'intégrase est un objectif essentiel.

Divers groupes à travers le monde ont développé des inhibiteurs de l'intégrase, certaines de ces molécules présentant des activités inhibitrices *in vitro* submicromolaires, comme la quercétagénine. D'autres composés ont présenté des activités prometteuses comme les esters phényléthyliques de l'acide caféique, le cosalane, la 5,8-dihydroxy-1,4-naphtoquinone, la cucurmine, la 1,10-phénanthroline, la primaquine, la chloroquine, certains dérivés de la podophyllotoxine ou encore des esters bis-galliques. Les activités de ces différents produits n'ont été cependant rapportés qu'*in vitro* et ces produits ne se sont pas révélés actifs *in vivo*.

Plus récemment des bis-caféates de l'acide quinique ont été décrits comme actifs *in vivo* mais selon des protocoles comportant la mise en contact préalable de la drogue avec le virus.

Les travaux des inventeurs dans ce domaine les ont amenés à étudier des dérivés de quinoléine et à mettre en évidence des propriétés anti-intégrase *in vitro* ainsi qu'*in vivo*, ces propriétés s'accompagnant d'une grande innocuité.

L'invention a donc pour but de fournir de nouveaux dérivés de quinoléine capables notamment d'inhiber l'activité intégrase de VIH *in vitro* et *in vivo*.

Elle vise également un procédé de synthèse de ces dérivés de mise en oeuvre aisée à l'échelle industrielle.

L'invention a également pour but la mise à profit des propriétés anti-intégrase de ces dérivés pour l'élaboration de médicaments.

Les dérivés selon l'invention sont **caractérisés en ce qu**'ils répondent à la formule générale II ;

Les dérivés selon l'invention sont **caractérisés en ce qu**'ils répondent à la formule générale II : 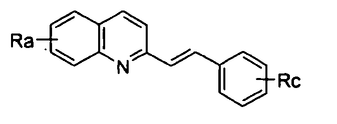 dans laquelle :
- Ra représente deux substituants dont l'un est un groupe -OH et l'autre est choisi parmi un groupe -(CH₂)ₙ -Y ou -CH=CH-Y,
   où Y représente un atome d'halogène, un radical -OH, -OR, -COH, -COR, -COOH, -COOR, -CONH₂, -CON (Rx,Ry), -CH=NOH, -CO-CH=NOH, -NH₂, -N (Rx, Ry), -NO₂, -PO(OR)₂, -SH₂, -SR, -SO₂R, -SO₂NHR, -CN, ou Z(R_{c}),
   où R représente un radical alkyle de 1 à 8 atomes de carbone, Rₓ et R_{y}, identiques ou différents, représentent un radical alkyle de 1 à 5 atomes de carbone et n est nul ou un entier de 1 à 5, Z représente un radical phényle,
- Rc représente deux ou trois substituants -OH, ainsi que les sels pharmaceutiquement acceptables de ces dérivés, leurs formes diastéréoisomères et leurs formes énantiomères.

"Halogène" désigne un atome de fluor, de chlore, de brome ou un groupe tritralogéno-méthyle, en particulier trichlorométhyle. "Alkyle" sans autre précision désigne un radical de 1 à 5 atomes de carbone.

Rₐ représente un groupe choisi parmi -CH=CH-COOH, -CH=CH-COOR, -CH=CH -COH, -CH=CH-COR, -CH=CH-CONH₂, -CON(Rₓ, R_{y}), et -CH=CH-Z(R_{c})

Des produits selon l'invention présentent la formule II
dans laquelle

-Rₐ représente deux substituants dont l'un est un groupe -OH et l'autre est choisi parmi OH, COOH.

-R_{c} représente deux ou trois substituants -OH.

Des produits particulièrement avantageux de l'invention comprennent, l'acide 8-hydroxy-2-[2-[(3,4-dihydroxyphényl)éthényl]] 7-quinoléine carboxylique, le sel de sodium de l'acide 8-hydroxy-2-[2-[(3,4-dihydroxyphényl) éthényl]] 7-quinoléinecarboxylique, la 7-cyano-8-hydroxy-2-[2-[(3,4-dihydro-xyphényl)éthényl]] quinoléine, l'acide 8-hydroxy-2-[2-[(3,4,5-trihydroxyphényl)éthényl]] 7-quinoléine carboxylique,

L'invention vise également un procédé de synthèse des dérivés définis ci-dessus.

Ce procédé est **caractérisé en ce qu**'il comprend - la réaction d'une quinaldine de formule III avec un dérivé aromatique ou hétéroaromatique de formule IV portant les groupes de blocage appropriés:

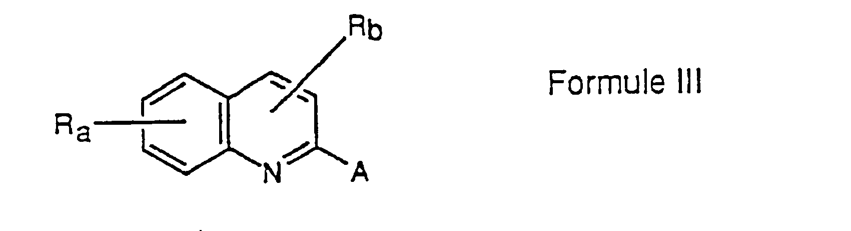

B-Z(R_{C}) Formule IV

dans lesquelles A et B représentent des groupes réactifs capables d'engendrer le groupe X, tel que défini ci-dessus, Rₐ, R_{c} présentant les significations données en rapport avec la formule II, mais comportant des groupes de blocage, et Rb=H, Z=phényle, et
- l'élimination des groupements protecteurs.

Selon un mode de réalisation de l'invention, pour obtenir les dérivés de quinoléine,
- on a recours à une condensation de type Perkin, entre une quinaldine de formule V et un dérivé aromatique ou hétéroaromatique de formule VI portant les groupes de blocage appropriés :

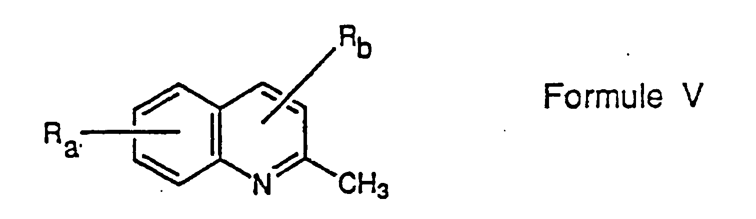

OHC-Z(R_{C}) Formule VI

dans lesquelles les différents substituants ont les significations données en rapport avec la formule II, mais comportent des groupes de blocage, et Rb=H et Z=phényle;
- on élimine les groupements protecteurs.

On opère à reflux dans un mélange pyridine-eau durant environ 2 heures à 3 jours.

Pour préparer par exemple des dérivés répondant à la formule II, on fait avantageusement réagir une quinaldine de formule V dans laquelle Rₐ représente au moins un substituant choisi parmi le groupe -OH, ou -COOH, ou une oxime, et de préférence deux substituants, dont l'un est un groupe -OH, et l'autre présente l'une des significations ci-dessus, avec un acétoxybenzaldéhyde de formule VII : 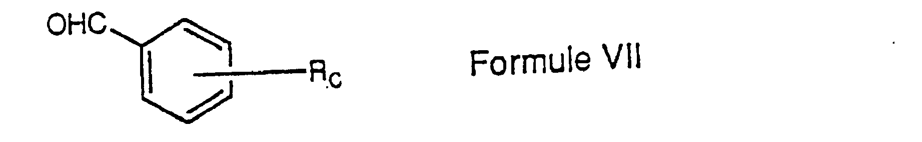 dans laquelle R_{c} représente au moins deux groupes -OH bloqués par des groupes protecteurs.

L'étape de condensation conduit à la formation de dérivés de quinoléine comportant une double liaison, et peuvent être traités si on le souhaite, selon les techniques classiques.

Les groupes de blocage sont éliminés par hydrolyse.

L'étude des propriétés biologiques des dérivés de l'invention a mis en évidence une activité inhibitrice vis-à-vis de l'intégrase. de VIH, et de l'enzyme EcoRI *in vitro*. Les expérimentations effectuées *in vivo* ont de plus montré leur effet inhibiteur de la réplication de VIH et l'absence d'effet sur les phases tardives de la réplication de VIH. Ces résultats revêtent donc un intérêt majeur pour le traitement d'une infection par ce virus, d'autant plus que les études de toxicité ont mis en évidence la grande innocuité de ces dérivés.

L'invention vise donc des compositions pharmaceutiques **caractérisées en ce qu**'elles comprennent une quantité efficace d'au moins un dérivé tel que défini ci-dessus, en association avec des véhicules pharmaceutiquement acceptabl es.

Ces compositions sont avantageusement utilisées en combinaison avec d'autres médicaments anti-VIH, en particulier des médicaments dotés d'un effet inhibiteur vis-à-vis de la réverse transcriptase et/ou de la protéase.

Les posologies et les modes d'administration seront adaptés en fonction du traitement mono-, bi-ou trithérapie utilisé.

L'invention vise également l'utilisation des dérivés définis ci-dessus en tant que réactifs biologiques utilisables en particulier pour des études de mécanismes concernant l'infection virale.

L'invention vise également un composé de formule (I) : 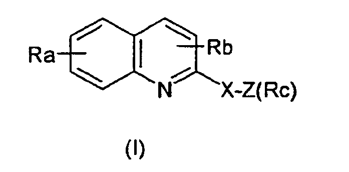 dans laquelle
- Ra, Rb et Rc identiques ou différents les uns des autres, représentent un ou plusieurs substituants, eux-mêmes identiques ou différents, occupant une position quelconque sur les cycles, et étant choisis parmi, un groupe -(CH,)ₙ -Y ou -CH=CH-Y, où Y représente un atome d'halogène, un radical -OH, -OR, -COH, -COR,
- COOH, -COOR, -CONH₂, -CON (Rx,Ry),-CH=NOH, -CO-CH=NOH, -NH₂, -N(Rx, Ry), -NO₂, -PO(OR)₂, -SH₂,-SR,-SO₂R, -SO₂NHR, -CN, ou Z(R_{c}), où R représente un radical alkyle de 1 à 8 atomes de carbone, Rₓ et R_{y}, identiques ou différents, représentent un radical alkyle de 1 à 5 atomes de carbone,
- Z représente un radical phényle et n est nul ou un entier de 1 à 5,
   Rb pouvant représenter en outre un atome d'hydrogène, et lorsque Y représente un groupe -COOH ou -COOR dans Rc, Z comporte au moins 3 substituants ou le noyau quinoléine est trisubstitué, et lorsque Y représente un groupe -CHO, Rc représente un groupe -(CH₂)ₙ-Y, avec n=1 à 5, X représente une double liaison éthylénique,
ou un groupe -(CH₂)ₙ- où n est un entier de 1 à 5, ou un groupe -CH(Rd)-CH(Re)-, Rd et Re, identiques ou différents, représentant un atome d'hydrogène, d'halogène, un groupe hydroxy ou époxy, ou un groupe -(CH₂)_{n'}-O-C(O)-(CH₂)ₘ-, -(CH₂)_{n'}-C(O)-(CH₂)ₘ-, -(CH₂)_{n'}-O-(CH₂)ₘ, -(CH₂)_{n'}-N(Q)-(CH₂)ₘ- ou -(CH₂)_{n'}-S(O)ₜ-(CH₂)ₘ-;
- où n' est un entier de 0 à 8, m est un entier de 0 à 8, t est nul ou un entier égal à 1 ou 2, et Q représente un atome d'hydrogène, un radical alkyle ou phényle,
   ainsi que les sels pharmaceutiquement acceptables de ces dérivés, leurs formes diastéréoisomères et leurs formes énantiomères, comme médicament antiviral.

Lesdits composés sont notamment utiles pour inhiber l'intégrase du VIH et/ou prévenir ou traiter le VIH.

Préférentiellement, lesdits composés de formule (I) sont choisis parmi les composés de formule (II) tels que définis plus haut.

D'autres caractéristiques et avantages de l'inventicn sont donnés dans les exemples qui suivent relatifs à la synthèse de dérivés de formule XII. 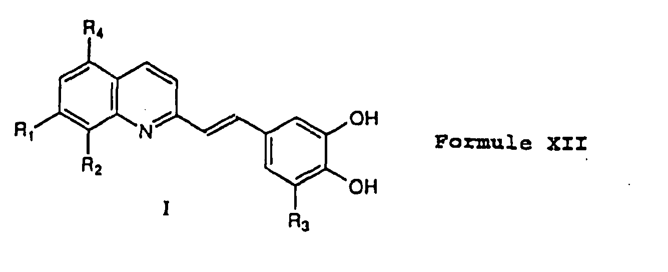 et à l'étude des propriétés anti-virales de dérivés répondant à cette formule.

### I Synthèse de dérivés de l'invention

### Exemple 1: 2-[2-[(3,4-dihydroxyphényl)éthényl]] quinoléine (I, R₁ = R₂ = R₃ = R₄ = H)

*1^{ère} étape*: préparation de la 2-[(3,4-diacétoxyphényl)éthényl] quinoléine. Un mélange de quinaldine (0,90 g, 6,3 mmol) et de 3,4-diacétoxybenzaldéhyde (1,15 g, 7,0 mmol) dans 10 ml d'anhydride acétique est porté à reflux pendant 12 h. Après refroidissement à 20 °C le mélange réactionnel est concentré sous pression réduite et le résidu chromatographié sur colonne de silice en éluant par un mélange (cyclohexane / acétate d'éthyle: 50 / 50) pour donner 2,08 g (95 %) de 2-[(3,4-diacétoxyphényl)éthényl] quinoléine qui est utilisé sans autre purification dans l'étape suivante.

*2^{ème} étape :* préparation du 2-[(3,4-dihydroxyphényl)éthényl] quinoléine. A une solution de l'ester précédent (étape 1) (2,00 g, 5,76 mmol) dans la pyridine (20 ml) on additionne 8 ml d'eau. Après 3 heures de reflux, le mélange est ramené à 20 °C, et 20 ml de dichlorométhane sont ajoutés. Les phases sont séparées et la phase aqueuse extraite au dichlorométhane. Les phases organiques rassemblées sont séchées sur sulfate magnésium, et concentrées sous pression réduite pour donner un solide jaune qui est recristallisé dans l'isopropanol. (1,51 g, 96 %)
F 246-251 °C
IR (KBr, cm-¹) n 3536, 1602.
RMN ¹H( DMSO d6, 200 MHz) d: 6,85 (d, *J* = 8,8 Hz, 1H); 7,10 (dd, *J* = 8,0, 1.0 Hz, 1H) 7,20 (m, 2H); 7,60 (t, *J* = 6,8 Hz, 1H); 7,70-8,10 (m, 5H); 8,35 (d, *J* = 8,8 Hz, 1H); 9,30 (s large, 2H).
RMN ¹³C ( DMSO d6, 50 MHz) d: 114,1; 116,0; 119,9 (2C); 125,4; 125,9; 126,9; 127,9 (2C); 128,6; 129,8; 134,8; 136,4; 145,7; 146,9; 147,8; 156,3.

### Exemple 2: 8-hydroxy-2-[2-[(3,4-dihydroxy-phényl)éthényl]] quinoléine (I, R₁ = H, R₂ = OH, R₃ = H, R₄ = H)

*1^{ère} étape*: préparation de la 8-acétoxy-2-[(3,4-diacétoxyphényl)éthényl] quinoléine. Un mélange de 8-hydroxyquinaldine (2,23 g, 14 mmol) et de 3,4-diacétoxybenzaldéhyde (2,6 g, 12 mmol) dans 30 ml d'anhydride acétique est porté à reflux pendant 12 h. Après refroidissement à 20 °C le mélange réactionnel est concentré sous pression réduite, repris à l'éther et filtré. Le résidu solide est lavé plusieurs fois à l'éther pour donner 3,5 g (61 %) de 8-acétoxy-2-[(3,4-diacétoxyphényl)éthényl] quinoléine qui est utilisé sans autre purification dans l'étape suivante.

*2^{ème} étape:* préparation de la 8-hydroxy-2-[(3,4-dihydroxyphényl)éthényl] quinoléine. A une solution de l'ester précédent (étape 1) (1,40 g, 3,4 mmol) dans la pyridine (10 ml) on additionne 5 ml d'eau. Après 3 heures de reflux, le mélange est ramené à 20 °C, et dilué par 30 ml d'eau. Le mélange réactionnel est alors abandonné à 20 °C. Après 12 h les cristaux obtenus sont filtrés, lavés à l'eau, l'éthanol puis à l'éther. Après recristallisation dans le xylène on obtient 0,6 g (63 %) de cristaux jaunes
F 232-235 °C
IR (KBr, cm-¹) n 3410, 1589.
RMN ¹H ( acétone d₆, 200 MHz) d: 6,87 (d, *J* = 8, 1 Hz, 1H); 7,05-7,10 (m, 2H); 7,21 (d, *J* = 16,2 Hz, 1H); 7,22 (s large, 1H); 7,72 (d, *J* = 8,7 Hz, 1H); 7,30-7,40 (m, 2H);7.91 (d, *J* = 16,2 Hz, 1H); 8,23 (d, *J* = 8,7 Hz, 1H), 8,20-8,70 (massif, 3H).
RMN ¹³C ( DMSO d6, 50 MHz) d: 111,2; 114,0; 116,0; 117,7; 119,8; 120,8; 124,7; 126,7; 127,5; 128,2; 135,1; 136,4; 138,2; 145,7; 146,8; 152, 9; 154,1.

### Exemple 3: acide 8-hydroxy-2-[2-[(3,4-dihydroxyphényl)éthényl]] 7-quinoléine carboxylique

### (I, R₁ = CO₂H, R₂ = OH, R₃ = H, R₄ = H)

Un mélange d'acide 8-hydroxy-7-quinaldine carboxylique (1,20 g, 6 mmol) et de 3,4-diacétoxybenzaldéhyde (1,8 g, 8 mmol) dans 15 ml d'anhydride acétique est porté à reflux pendant 12 h.

L'acide 8-hydroxy-7- quinaldine carboxylique est synthétisé en opérant selon Meek et al dans J. Chem. Engineering data, 1969, 14, 388-391.

Après refroidissement à 20 °C le mélange réactionnel est concentré sous pression. Le résidu obtenu est dissous dans la pyridine (20 ml) on additionne 8 ml d'eau. Après 3 heures de reflux, le mélange est ramené à 20 °C, et dilué par 20 ml d'eau. Le mélange est extrait au dichlorométhane, la phase organique est écartée et la phase aqueuse abandonnée à 20 °C. Après 12 h les cristaux obtenus sont filtrés, lavés à l'eau, l'éthanol puis à l'éther puis séchés sous vide pour donner 0,96 g (50 %) de cristaux rouge vif.
F ≥ 300 °C
IR (KBr, cm-¹) n 3091, 1667, 1589.
RMN ¹H (DMSO d6, 200 MHz) d: 6,80 (d, *J* = 8,1 Hz, 1H); 7,04 (dd, *J* = 8,2; 1,5 Hz, 1H); 7,10-7,18 (m, 2H); 7,45 (d, *J* = 16,2 Hz, 1H); 7,78-7,90 (m, 2H), 8,15 (d, *J* = 8,8 Hz, 1H); 8,48 (d, *J* = 8,8 Hz, 1H); 9,22 (s, large, 1H); 9,52 (s, large, 1H).
RMN ¹³C ( DMSO d6, 50 MHz) d: 112,8; 113,2; 114,2, 116,0; 120,2; 120,7; 120,9; 127,3; 127,5; 130,5; 135,2; 139,2; 140,0; 145,7; 148,0; 152,8; 160,8; 170,6.

### Exemple 4: sel de sodium de l'acide 8-hydroxy-2-[2-[(3,4-dihydroxyphényl) éthényl]] 7-quinoléinecarboxy-lique (I, R₁ = CO₂Na, R₂ = OH, R₃ = H, R₄ = H)

L'acide (I, R₁ = CO₂H, R₂ = OH, R₃ = H) (0,1 g, 0,3 mmol) est additionné par portion à une solution de soude 0,1 M maintenue sous atmosphère d'azote. Le mélange est alors lyophylisé (-40 à +40 °C) pour donner 0,11 g de sel de sodium (quantitatif) sous forme d'un solide gris brun.
IR (KBr, cm-¹) n 3436, 1598, 1561.
RMN ¹H (DMSO d6, 200 MHz) d: 4,5-5,5 (massif, 2H); 6,74 (d, *J* = 7,8 Hz, 1H); 6, 95 (m, 2H), 7,13 (s, 1H), 7,24 (d, *J* = 16,4 Hz, 1H); 7,49 (d, *J* = 16,4 Hz, 1H); 7,75 (d, *J* = 8,4 Hz, 1H); 7,84 (d, *J* = 8,6 Hz, 1H); 8,08 (d, *J* = 8,6 Hz, 1H); 12,6 (s large, 1H).

RMN ¹³C ( DMSO d6, 50 MHz) d: 111,9; 113,7; 114,9; 115,8; 119,2; 119,5; 126, 0; 127,0; 127,5; 129,9; 133,6; 136,0; 141,1; 146,2; 147,7; 154,0; 163,7; 171,6.

### Exemple 5: 7-cyano-8-hydroxy-2-[2-[(3,4-dihydroxyphényl)éthényl]] quinoléine (I, R₁ = CN, R₂ = OH, R₃ = H, R₄ = H)

*1^{ère} étape*: préparation de l'oxime du 8-hydroxy-7-quinaldine carbaldéhyde.

A une solution de 8-hydroxy-7-quinaldine carbaldéhyde (1g, 53 mmol) dans l'acide acétique (20 ml), sont additionnés successivement 0,73 g (10 mmol) de chlorhydrate d'hydroxylamine puis 0,87 g (0,01 mmol) d'acétate de sodium. Le 8-hydroxy-7quinaldine carbaldéhyde est synthétisé en opérant selon Przystal et al, J. Heterocycl. Chem, 1967, 4, 131-2.

Le mélange est chauffé à 100 °C durant 1 h puis concentré sous pression réduite. Le résidu est repris par 20 ml d'eau et extrait au dichlorométhane. Les phases organiques sont rassemblées, séchées sur MgSO₄ et concentrées pour donner 0,7 g de l'oxime attendue sous forme d'un solide jaune (65 %).
F = 171-175 °C
IR (KBr, cm-¹) n 3200-2800, 1643, 1614, 1563.

L'analyse des spectres de RMN révèle la présence de deux isomères syn/anti, seul l'isomère majoritaire est décrit ci-dessous.

RMN ¹H (DMSO d6, 200 MHz) d: 2,68 (s, 3H); 7,32 (d, *J* = 8,6 Hz, 1H); 7,42 (d, *J* = 8,3 Hz, 1H); 7,70 (d, *J* = 8,6 Hz, 1H); 8,16 (d, *J* = 8,4 Hz, 1H); 8,50 (s, 1H)
RMN ¹³C (DMSO d6, 50 MHz) d: 24,7; 115,2, 117,9; 122,9; 123,2; 127,2; 136,2; 138,1; 144,7, 150,8; 157,4. *2^{ème} étape*:: préparation du 7-cyano-8-acétoxy-2-[(3,4-diacétoxyphényl)éthényl] quinoléine. Un mélange de l'oxime du 8-hydroxy-7-quinaldine carbaldéhyde (0,60 g, 2,9 mmol) et de 3,4-diacétoxybenzaldéhyde (0,78 g, 3,4 mmol) dans 10 ml d'anhydride acétique est porté à reflux pendant 12 h. Après refroidissement à 20 °C le mélange réactionnel est concentré sous pression réduite. Le résidu obtenu est chromatographié sur colonne de silice en éluant par un mélange (cyclohexane / acétate d'éthyle: 50 / 50) pour donner 0,77 g (62 %) de solide jaune clair.

IR (KBr, cm-¹) n 2941, 2223, 1782, 1600, 1504.

RMN ¹H (CDCl₃, 200 MHz) d: 2,30 (s, 3H); 2,31 (s, 3H); 2,60 (s, 3H); 7,00-7,70 (m, 8H); 8,09 (d, *J* = 8,6 Hz, 1H).
RMN ¹³C (CDCl₃, 50 MHz) d: 20,7 (2 C); 20,8; 106,8; 115,3; 122,0; 122,6; 123,8; 125,7; 126,2; 129,0; 130,7; 134,3; 134,9; 136,4; 140,5; 142,4; 142,5; 151,6; 156,8; 168,1 (2C); 68,5.
*3^{ème} étape:* préparation du 7-cyano-8-hydroxy-2-[(3,4-dihydroxyphényl)éthényl] quinoléine. A une solution du triester précédent (étape 2) (0,60 g, 1,4 mmol) dans la pyridine (10 ml) on additionne 5 ml d'eau. Après 3 heures de reflux, le mélange est ramené à 20 °C, et dilué par 20 ml d'eau. Les phases sont séparées et la phase aqueuse extraite au dichlorométhane. Les phases organiques rassemblées sont séchées sur sulfate magnésium, et concentrées sous pression réduite pour donner un solide jaune qui est recristallisé dans l'isopropanol (0,3 g, 71 %).
F 239-244 °C
IR (KBr, cm-¹) n 3411, 2194, 1603, 1554.
RMN ¹H (DMSO d6, 200 MHz) d: 6,79 (d, *J* = 8,2 Hz, 1H); 7,00 (d, *J* = 8,4 Hz, 1H); 7,05-7,25 (m, 2H); 7,41 (d, *J* = 8,5 Hz, 1H); 7,54 (d, *J* = 8,5 Hz, 1H); 7,87 (d, *J* = 8, 6 Hz, 1H); 8,04 (d, *J* = 16,2 Hz, 1H); 8,32 (d, *J* = 8,6 Hz, 1H); 8,80-9,50 (massif, 3H).
RMN ¹³C ( DMSO d6, 50 MHz) d: 114,0; 115,9; 117,1; 118,5; 119,9; 123,3; 123,7; 123,9; 126,5; 127,8; 129,6; 136,7; 137,6; 145,6; 147,0; 149,6; 155,7; 158,2.

### Exemple 6: acide 8-hydroxy-2-[2-[(3,4,5-trihydroxyphényl)éthényl]] 7-quinoléine carboxylique

### (I, R₁ = CO₂H, R₂ = R₃ = OH, R₄ = H)

Un mélange d'acide 8-hydroxyquinaldine carboxylique (0,23 g, 1,13 mmol) et de 3,4,5-triacétoxybenzaldéhyde (0,32 g, 1,16 mmol) dans 7 ml d'anhydride acétique est porté à reflux pendant 48 h. Après refroidissement à 20 °C le mélange réactionnel est concentré sous pression. Le résidu obtenu est dissous dans la pyridine (7 ml) on additionne 3 ml d'eau. Après 3 heures de reflux, le mélange est ramené à 20 °C, et dilué par 20 ml d'eau. Le mélange est extrait au dichlorométhane, la phase organique est écartée et la phase aqueuse abandonnée à 20 °C. Après 12 h les cristaux obtenus sont filtrés, lavés à l'eau, l'éthanol puis à l'éther puis séchés sous vide pour donner 0,15 g (40 %) de cristaux rouge vif.
F ≥ 300 °C
IR (KBr, cm-¹) n 3600-2400, 1630, 1585.
RMN ¹H (DMSO d6, 200 MHz) d: 3,50-5,50 (massif, 4H); 6,68 (s, 2H); 7,16 (d, *J* = 8,4 Hz, 1H); 7,44 (d, *J* = 16,1 Hz, 1H); 7,80 (d, *J* = 16,1 Hz, ¹H); 7, 84 (d, *J* = 8,4 Hz, 1H); 8,21 (d, *J* = 8,8 Hz, 1H); 8,51 (d, *J* = 8,8 8 Hz, 1H); 9,19 (s, largue, 1H).
RMN ¹³C (DMSO d6, 50 MHz) d: 107,4 (2C); 112,9; 113,2; 120,0; 120,8; 126,2; 127,8; 130,5; 134,9; 136,3; 140,1; 140,4; 146,4 (2C); 152,7; 161,0; 170,6.

### Exemple 7: acide 2-[2-[(3,4,-dihydroxyphényl)éthényl]] 5,7-quinoléinedicarboxylique

### (R1 = R4 = CO₂H, R₂ = R₃ = H)

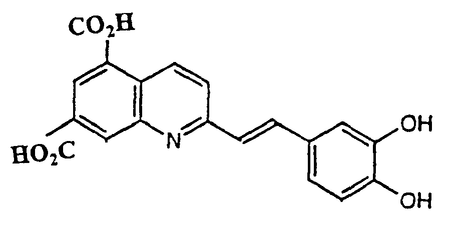

***1ère étape*: préparation de l'acide 2-méthyl-5,7-quinoléinedicarboxylique**: Une solution d'acide 5-amino-1,3-benzènedicarboxylique (5,3 g, 29 mmol) dans 60 mL d'acide chlorhydrique 6 *N* est portée au reflux. 2,6 mL (32 mmol) de crotonaldéhyde sont additionnés goutte à goutte sur une période d'une heure et le chauffage est maintenu pendant une heure. Après refroidissement à 20°C, le mélange est extrait à l'éther. La phase organique est écartée et la phase aqueuse est basifiée par une solution d'ammoniaque à 30%. Le précipité est filtré, lavé à l'eau puis à l'éther et séché sous vide pour fournir 2,5 g d'acide 2-méthyl-5,7-quinoléinedicarboxylique (Rdt; 37 %)
RMN ¹H (CD₃OD, 200 MHz) δ: 1,30 (s, 3H); 6,40 (d, *J* = 8,8 Hz,1H); 7,20 (s,2H) 8,25 (d, *J* = 8,8 Hz, 1H).

***2ème étape:* préparation de l'acide 2-[2-[(3,4-dihydroxyphényl)éthényl]] 5,7-quinoléinedicarboxylique:** Un mélange d'acide 2-méthyl-5,7-quinoléinedicarboxylique (0,60 g, 2,6 mmol) et de 3,4-diacétoxybenzaldéhyde
(0,71 g, 3,1 mmol) dans 10 mL d'anhydride acétique est porté à reflux pendant 48 h. Après refroidissement à 20°C le mélange réactionnel est concentré sous pression réduite et le résidu est repris dans la pyridine (10 mL). Le mélange est porté au reflux, puis 3 mL d'eau sont additionnés. Après 3 heures de reflux, le mélange est ramené à 20°C, et 25 mL d'une solution aqueuse d'acide acétique sont ajoutés. Le mélange est extrait au dichlorométhane. Les phases organiques rassemblées, séchées sur sulfate magnésium, et concentrées sous pression réduite pour donner un solide rouge qui est recristallisé dans l'isopropanol.
RMN ¹H (DMSO d6, 200 MHz) δ: 6,78(d,J = 8,2 Hz, 1H); 7,00-7,30 (m, 3H); 7,74 (d, *J* = 16,2 Hz,1H);
8 , 03 (d, *J* = 9,2 Hz,1H); 8,56 (s,1H); 8,64 (s,1H);
9,22 (d, *J* = 9,2 Hz, 1H).

### II - Etude des propriétés antivirales dirigées contre l'intégrase du VIH-1 de dérivés selon l'invention

Sont décrites ci-après en exemple les activités anti-intégrase et antivirales des composés suivants:
**-Composé 1: acide 8-hydroxy-2-[2-[(3,4-dihydroxyphényl)éthényl]] 7-quinoléine carboxylique**

(R₁ = CO₂H, R₂ = OH, R₃ = R₄ = H)

**-Composé 2 : sel de sodium de l'acide 8-hydroxy-2-[2-[(3,4-dihydroxyphényl) éthényl]] 7-quinoléine-carboxylique** (R₁ = CO₂Na, R₂ = OH, R₃ = R₄ = H)
**-Composé 3: acide 8-hydroxy-2-[2-[(3,4,5-trihydroxyphényl)éthényl]] 7-quinoléine carboxylique**

(R₁ = CO₂H, R₂ = R₃ = OH, R₄ = H)

**-Composé 7: acide 2-[2-[(3,4-dihydroxyphényl)éthényl]] 5,7-quinoléinecarboxylique**

(R₁=R₄ = COOH; R₂ = R₃=H)

### Exemple 1: Inhibition de l'activité in vitro de l'intégrase du VIH-1

L'activité de l'intégrase du VIH-1 est mesurée par les trois tests suivants:
1- L'activité endonucléolytique de la protéine est testée sur un oligonucléotide double-brins de 21 paires de bases, radiomarqué à l'extrémité 5'. L'activité de l'intégrase se traduit par l'élimination du dinucléotide à l'extrémité 3'.
2- Le test de transfert de brins est réalisé avec un oligonucléotide double-brins de 21 paires de bases mimant l'extrémité de l'ADN viral dont le dinucléotide 3' terminal a été supprimé. L'activité de la protéine se traduit par l'insertion covalente de cet oligonucléotide dans un oligonucléotide homologue.
3- Le test de désintégration est réalisé avec un substrat mimant la structure de l'ADN viral intégré. On mesure la quantité d'ADN excisé par l'intégrase. Ce dernier test quantifie uniquement l'activité catalytique de la protéine à l'exclusion de son activité de fixation sur l'ADN.

Les composés 1 et 3, selon la présente invention, inhibent les trois activités de l'intégrase du VIH-1. L'inhibition de la désintégration suggère que ce sont des inhibiteurs de l'activité catalytique de l'enzyme. Les degrés d'inhibition dans les trois tests pour chacun des composés sont comparables ce qui montre que les composés 1 et 3 ne perturbent pas la fixation de l'intégrase sur son substrat. Le tableau I indique les activités inhibitrices des composés selon l'invention. L'inhibition est exprimée en concentration nécessaire pour bloquer 50% de l'activité de l'intégrase du VIH-1.

**Tableau I: Inhibition de l'activité in vitro de l'intégrase du VIH-1 par les composés 1 et Composé 3 selon la présente invention**

| | coupure endonucléolytique | transfert de brins | désintégration |
|---|---|---|---|
| Composé 1 | 0,9 µM | 0,9 µM | 0,5 µM |
| Composé 3 | 0,32 µM | 0,31 µM | 0,056 µM |

**Nota:** Le composé 2 qui est le sel de sodium de l'acide libre 1 possède la même activité anti-intégrase que celui-ci dans les tests d'activité *in vitro*. Le composé 2 est soluble dans l'eau à 10 mM tandis que le composé 1 est soluble dans le DMSO.

### Exemple 2: Inhibition de l'enzyme de restriction EcoRI

Le site catalytique de l'intégrase du VIH-1 est très proche de celui de l'enzyme de restriction EcoRI. Les composés qui bloquent à la fois les deux protéines sont donc des inhibiteurs catalytique de l'intégrase.

Le composé 1 selon la présente invention inhibe la coupure d'un plasmide linéarisé par l'enzyme EcoRI. Le test est réalisé de la façon suivante: Le plasmide pSP65 est linéarisé et radiomarqué à son extrémité 5' par la polynucléotide kinase. Le plasmide linéarisé est incubé 4 heures en présence de 0,1 unités de l'enzyme EcoRI. L'activité de l'enzyme est suivie par l'apparition des produits de coupure sur gel d'agarose 1,2%.

En présence du composé 1 selon la présente invention, la coupure par EcoRI est fortement inhibé. Ce composé est donc un inhibiteur de l'activité catalytique de la protéine.

### Exemple 3: absence d'inhibition de la transcritpion inverse par le composé 1

La spécificité des composés selon l'invention pour l'intégrase est estimée par un test d'activité sur l'enzyme transcriptase inverse du virus VIH-1. Ce test est réalisé de la façon suivante: Les particules virales d'un surnageant de culture des cellules CEM (lignée lymphocytaire établie) sont concentrées par centrifugation à 30 000RPM. Ces particules virales sont lysées dans un détergent non ionique et leur activité transcriptase inverse est testée sur un substrat formé d'un primer oligo(dG) hybridé sur une matrice polyrC. En présence de nucléotide dG radiomarqué, l'activité transcriptase se traduit par la formation de polynucléotide marqué précipitable dans l'acide trichloroacétique. On vérifie qu'un didéoxynucléotide ddG inhibe la formation d'acido-précipitable.

En présence du composé 1, on n'observe aucune inhibition de la transcription inverse d'une matrice polyrC. Ce résultat traduit la bonne sélectivité du composé 1 pour l'intégrase virale.

### Exemple 4: inbibition de la réplication du Virus de l'Immunodéficience Humaine (VIH-1)

Le test d'activité consiste à mettre en contact des cellules d'une lignée lymphocytaires établie, les cellules CEM, avec un surnageant de cellules infectées contenant les virions infectieux. Le test est réalisé de la façon suivante: Les cellules CEM, cultivées en suspension dans du milieu RPMI complémenté avec 10% de sérum de veau foetal, sont infectées par un surnageant viral avec une multiplicité d'infection de 0,5. Après 2 heures d'infection, les cellules sont lavées deux fois avec du milieu RPMI de façon à éliminer les particules virales résiduelles. Enfin, les cellules sont replacées dans du milieu RPMI contenant le composé selon l'invention. La charge virale est évaluée après 72 heures de culture. Celle-ci est quantifiée des deux manières suivantes:
1) La quantité de protéine virale p24 est déterminée par un test ELISA.
2) La charge en virus infectieux est estimée par infection des cellules HeLa β-gal CD4⁺ (cf paragraphe 4).

La toxicité des composés est testée par un test de biotransformation du MTT en formazan par les déshydrogénases mitochondriales cellulaires.

Dans les deux tests d'activité, le composé 1 selon la présente invention, possède un effet protecteur contre l'infection des cellules CEM par le VIH-1. Cet effet protecteur se traduit par une inhibition de la production de particules virales avec une efficacité 50% de 4 µM selon le test HeLaβ-gal et avec une efficacité 50% de 28 µM selon le test p24. Le composé 1 est dépourvu de toxicité à 100 µM, concentration maximale testée, sur les cellules CEM d'après le test MTT.

Le composé 2 bloque la production de particules virales, mesurées par le test ELISA p24, lors de l'infection des cellules CEM. Ce composé est également dépourvu de toxicité selon le test MTT, jusqu'à 100 µM.

Ces résultats sont résumés dans le tableau II.

Le composé 2 qui correspond au sel de sodium, de l'acide libre 1 présente le même effet antiviral mesuré par l'inhibition de la réplication dans les cellules CEM à 72 heures.

### Exemple 5: Absence d'inhibition des étapes tardives de la réplication du VIH-1 par le composé 1

Les composés selon la présente invention ont été testés sur les étapes tardives de la réplication du VIH-1. Le test est le suivant: On utilise les cellules ACH2 qui ont intégré l'ADN viral du VIH-1. Ces cellules n'expriment pas les protéines virales et ne produisent donc pas de virus à moins d'être activées par le TNF. Lorsque les cellules sont mises en présence de TNF, elles expriment du virus VIH à partir du provirus intégré. La production de particules virales est détectée 24 heures après activation. La charge virale dans le surnageant est quantifiée par test ELISA p24.

Aucun effet important n'a été détecté sur la production de particules virales à 24 heures lorque les cellules ACH2 sont traitées avec des concentrations du composé 1 jusqu'à 100 µM. On observe une légère inhibition au-delà de 50 µM mais la concentration inhibitrice 50% n'est pas atteinte à 100 µM.

**Tableau II: Effet antiviral du composé 1**

| | MTTⁱ | activité β-gal^{j} | test p24^{k} |
|---|---|---|---|
| Cellule CEM^{a} | >100 µM | 4 uM | 20 µM |
| Cellules ACH2^{b} | >100 µM | - | >100 µM |

**Tableau III: Effet antiviral du composé 7**

| | MIT¹ | activité β-gal^{j} | test p24^{k} |
|---|---|---|---|
| Cellule CEM^{a} | >100 µM | 1 µM | 4 µM |
| Cellules ACH2^{b} | >100 µM | | >100 µM |

| | | | |
|---|---|---|---|
| ^{a}effet antiviral global; ^{b}effet sur les étapes tardives ⁱToxicité par biotransformation; ^{j}test colorimètrique;^{k}test ELISA | | | |

En conclusion, les composés donnés en exemple inhibent sélectivement l'activité *in vitro* de l'intégrase du VIH-1 dans les trois tests utilisés. Le composé 1 qui est dépourvu de cytotoxicité jusqu'à 100 µM dans les modèles cellulaires utilisés, bloque la réplication du VIH en interférant avec une étape précoce du cycle de réplication. Etant donné l'absence d'effet sur la transcription inverse *in vitro,* cette étape est vraisembablement l'intégration du génome viral dans le génome de la cellule infectée.

## Revendications

1. Dérivés de quinoléine, **caractérisés en ce qu'**ils répondent à la formule générale II : 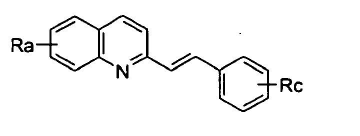 dans laquelle : - Ra représente deux substituants dont l'un est un groupe -OH et l'autre est choisi parmi un groupe -(CH₂)ₙ -Y ou -CH=CH-Y,
où Y représente un atome d'halogène, un radical -OH, -OR, -COH, -COR, -COOH, -COOR, -CONH₂, -CON (Rx,Ry), -CH=NOH, -CO-CH=NOH, -NH₂, -N(Rx, Ry), -NO₂, -PO(OR)₂, -SH₂, -SR, -SO₂R, -SO₂NHR, -CN, ou Z(R_{c}),
où R représente un radical alkyle de 1 à 8 atomes de carbone, Rₓ et R_{y}, identiques ou différents, représentent un radical alkyle de 1 à 5 atomes de carbone et n est nul ou un entier de 1 à 5, Z représente un radical phényle ; - Rc représente deux ou trois substituants -OH,
ainsi que les sels pharmaceutiquement acceptables de ces dérivés, leurs formes diastéréoisomères et leurs formes énantiomères.

2. Dérivés selon la revendication 1, dans lesquels Rₐ est un groupe choisi parmi -CH=CH-COOH, -CH=CH-COOR, -CH=CH-COH, -CH=CH-COR, -CH=CH-CONH₂,-CON(Rₓ, R_{y}) et -CH=CH-Z(R_{c}).

3. Dérivés selon la revendication 1 ou 2, **caractérisés en ce qu'**ils présentent la formule II 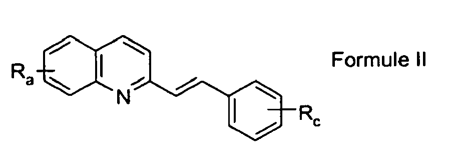 dans laquelle
- Rₐ représente deux substituants dont l'un est un groupe -OH et l'autre est choisi parmi OH, COOH, et
- R_{c} représente deux ou trois substituants -OH.

4. Dérivés de quinoléine **caractérisés en ce qu** il s'agit de l'acide 8-hydroxy-2-[2-[(3,4-dihydroxyphényl)éthényl]]7-quinoléine carboxylique, le sel de sodium de l'acide 8-hydroxy-2-[2-[(3,4-dihydroxyphényl) éthényl]]7-quinoléine carboxylique, la 7-cyano-8-hydroxy-2-[2-(3,4-dihydroxyphényl) éthényl]] quinoléine, l'acide 8-hydroxy-2-[2-[(3,4,5-trihydroxyphényl)éthényl]] 7-quinoléine carboxylique;

5. Procédé de synthèse de dérivés selon la revendication 1, **caractérisé ce qu'**il comprend - la réaction d'une quinaldine de formule III avec un dérivé aromatique ou hétéroaromatique de formule IV portant les groupes de blocage appropriés : 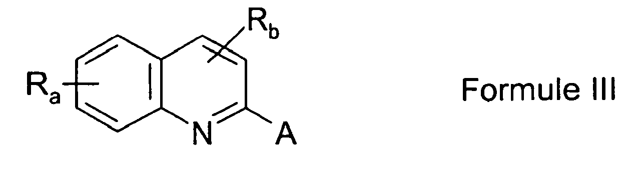
B-Z(R_{c}) Formule IV
dans lesquelles A et B représentent des groupes réactifs capables d'engendrer le groupe X, tel que défini ci-dessus, Rₐ, R_{c} présentant les significations données en rapport avec la formule **II,** mais comportant des groupes de blocage, et Rb=H, et Z=phényle ; et
- l'élimination des groupements protecteurs.

6. Procédé selon la revendication 5, **caractérisé en ce que**, pour préparer des dérivés de formule **II,**
- on fait réagir une quinaldine de formule V et un dérivé aromatique ou hétéroaromatique de formule VI portant les groupes de blocage appropriés, 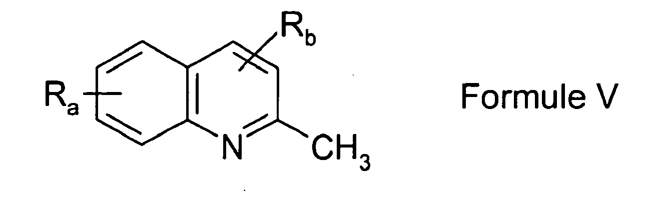
OHC-Z(R_{c}) Formule IV
dans lesquelles les différents substituants ont les significations données en rapport avec la formule II mais comportant des groupes de blocage, et Rb=H et Z=phényle ;
- on élimine ces groüpes de blocage.

7. Procédé selon la revendication 6, **caractérisé en ce que** pour préparer des dérivés de formule II selon la revendication 3, on fait réagir une quinaldine de formule V dans laquelle Rₐ représente au moins un substituant choisi parmi le groupe -OH, -COOH, ou une oxime, et de préférence deux substituants, dont l'un est un groupe -OH, et l'autre présente l'une des significations ci-dessus, avec un acétoxybenzaldéhyde de formule VII : 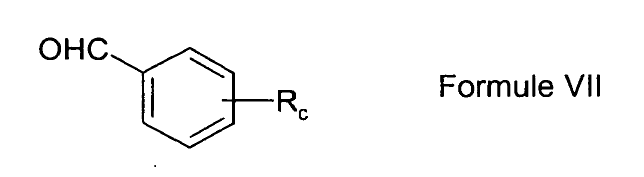 dans laquelle R_{c} représente au moins deux groupes -OH bloqués par des groupes protecteurs.

8. Compositions pharmaceutiques **caractérisées en ce qu'**elles comprennent en association avec des véhicules pharmaceutiquement acceptables une quantité efficace d'au moins un dérivé de quinoléine de formule (**II**) selon l'une quelconque des revendications 1 à 4.

9. Utilisation d'un composé de formule (**II**) selon l'une quelconque des revendications 1 à 4 comme réactif biologique.

10. Composé de formule (I) : 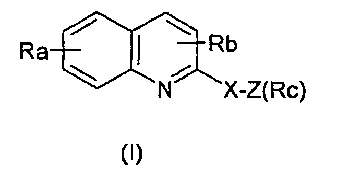 dans laquelle
- Ra, Rb et Rc, identiques ou différents les uns des autres, représentent un ou plusieurs substituants, eux-mêmes identiques ou différents, occupant une position quelconque sur les cycles, et étant choisis parmi un groupe -(CH₂)ₙ-Y ou -CH=CH-Y, où Y représente un atome d'halogène, un radical -OH, -OR, -COH, -COR, -COOH, -COOR, -CONH₂, -CON (Rx,Ry), -CH=NOH, -CO-CH=NOH, -NH₂, -N(Rx, Ry), -NO₂, -PO(OR)₂, -SH₂, -SR, -SO₂R, -SO₂NHR, -CN, ou Z(R_{c}),
où R représente un radical alkyle de 1 à 8 atomes de carbone, Rₓ et R_{y}, identiques ou différents, représentent un radical alkyle de 1 à 5 atomes de carbone, - Z représente un radical phényle et n est nul ou un entier de 1 à 5,
Rb pouvant représenter en outre un atome d'hydrogène,
et lorsque Y représente un groupe -COOH ou -COOR dans Rc, Z comporte au moins 3 substituants ou le noyau quinoléine est trisubstitué, et lorsque Y représente un groupe -CHO, Rc représente un groupe -(CH₂)ₙ-Y, avec n=l à 5, X représente une double liaison éthylénique, ou un groupe -(CH₂)ₙ- où n est un entier de 1 à 5, ou un groupe -CH(Rd)-CH(Re)-, Rd et Re, identiques ou différents, représentant un atome d'hydrogène, d'halogène, un groupe hydroxy ou époxy, ou un groupe -(CH₂)_{n'}-O-C(O)-(CH₂)ₘ-, -(CH₂)_{n'}-C(O)-(CH₂)ₘ-, -(CH₂)_{n'}-O-(CH₂)ₘ-, -(CH₂)_{n'}-N(Q)-(CH₂)ₘ-, ou -(CH₂)_{n'}-S(O)ₜ-(CH₂)ₘ-, où n' est un entier de 0 à 8, m est un entier de 0 à 8, t est nul ou un entier égal à 1 ou 2, et Q représente un atome d'hydrogène, un radical alkyle ou phényle, ainsi que les sels pharmaceutiquement acceptables de ces dérivés, leurs formes diastéréoisomères et leurs formes énantiomères,
**comme** médicament antiviral.

11. **Composé** selon la revendication 10 **pour inhiber** l'intégrase du VIH.

12. **Composé** selon la revendication 10 pour prévenir ou traiter le VIH.

13. **Composé** selon l'une quelconque des revendications 10 à 12 tel que défini selon l'une quelconque des revendications 2 à 4.

14. Utilisation d'un composé selon l'une quelconque des revendications 10 ou 13 pour la préparation d'un médicament pour prévenir ou traiter le VIH.

15. Combinaison comprenant un composé selon la revendication 10 ou 13 avec un autre médicament anti-VIH.

16. Combinaison selon la revendication 14 telle que ledit médicament anti-VIH est un inhibiteur de la réverse transcriptase et/ou de la protéase.

## Claims

1. Quinoline derivatives, **characterised in that** they correspond to the general formula II: 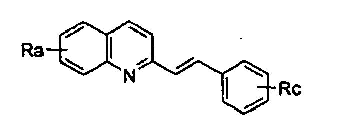 wherein:
- Ra represents two substituents of which one is a group -OH and the other is selected from a group -(CH₂)ₙ -Y or -CH=CH-Y, where Y represents a halogen atom, a radical -OH, -OR, -COH, -COR, -COOH, -COOR, -CONH₂, -CON (Rx,Ry),
- CH=NOH, -CO-CH=NOH, -NH₂, -N(Rx, Ry), -NO₂, -PO(OR)₂, -SH₂, -SR, -SO₂R,
- SO₂NHR, -CN or Z(R_{c}),
where R represents an alkyl radical of 1 to 8 carbon atoms, Rₓ and R_{y}, which are the same or different, represent an alkyl radical of 1 to 5 carbon atoms and n is zero or an integer from 1 to 5, Z represents a phenyl radical;
- Rc represents two or three substituents -OH, and also pharmaceutically acceptable salts of those derivatives, their diastereoisomeric forms and their enantiomeric forms.

2. Derivatives according to claim 1, wherein Rₐ is a group selected from
- CH=CH-COOH, -CH=CH-COOR, -CH=CH-COH, -CH=CH-COR, -CH=CH-CONH₂,
- CON(Rₓ, R_{y}) and -CH=CH-Z(R_{c}).

3. Derivatives according to claim 1 or 2, **characterised in that** they have the formula II 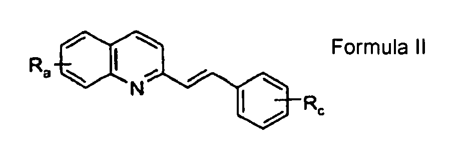 wherein
- Rₐ represents two substituents of which one is a group -OH and the other is selected from OH, COOH, and
- R_{c} represents two or three substituents -OH.

4. Quinoline derivatives, **characterised in that** they are 8-hydroxy-2-[2-[(3,4-dihydroxyphenyl)ethenyl]]7-quinoline carboxylic acid, 8-hydroxy-2-[2-[(3,4-dihydroxyphenyl)ethenyl]]7-quinoline carboxylic acid sodium salt, 7-cyano-8-hydroxy-2- [2-(3,4-dihydroxyphenyl) ethenyl]] quinoline, 8-hydroxy-2-[2- [(3,4,5-trihydroxyphenyl)ethenyl]] 7-quinoline carboxylic acid.

5. Process for the synthesis of derivatives according to claim 1, **characterised in that** it comprises
- reaction of a quinaldine of formula III with an aromatic or heteroaromatic derivative of formula IV carrying appropriate blocking groups: 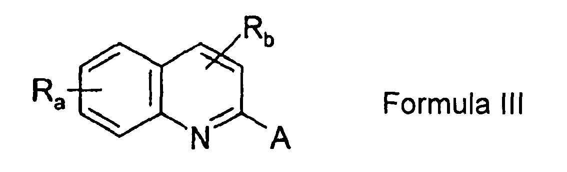
B-Z(R_{c}) Formule IV
wherein A and B represent reactive groups capable of producing the group X as defined hereinbefore, Rₐ and R_{c} have the meanings given in relation to formula II, but including blocking groups, and Rb=H and Z=phenyl; and
- removal of the protecting groups.

6. Process according to claim 5, **characterised in that**, in order to prepare derivatives of formula II,
- a quinaldine of formula V and an aromatic or heteroaromatic derivative of formula VI carrying appropriate blocking groups are reacted, 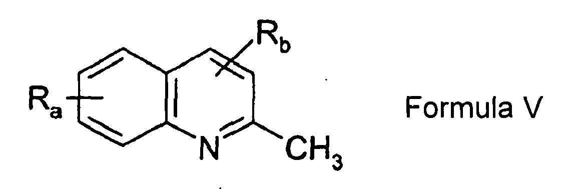
OHC-Z(R_{c}) Formula VI
wherein the various substituents have the meanings given in relation to formula II, but including blocking groups, and Rb=H and Z=phenyl;
- those blocking groups are removed.

7. Process according to claim 6, **characterised in that**, in order to prepare derivatives of formula II according to claim 3, a quinaldine of formula V, wherein Rₐ represents at least one substituent selected from the group -OH, -COOH or an oxime, and preferably two substituents of which one is a group -OH and the other has one of the above meanings, is reacted with an acetoxybenzaldehyde of formula VII: 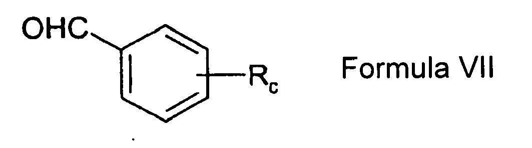 wherein R_{c} represents at least two groups -OH blocked by protecting groups.

8. Pharmaceutical compositions, **characterised in that** they comprise, in association with pharmaceutically acceptable carriers, an effective amount of at least one quinoline derivative of formula (II) according to any one of claims 1 to 4.

9. Use of a compound of formula (II) according to any one of claims 1 to 4 as a biological reagent.

10. Compound of formula (I): 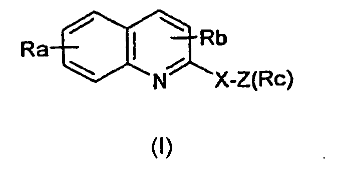 wherein
- Ra, Rb and Rc, which are the same or different from one another, represent one or more substituents, which are themselves the same or different, occupying any position on the rings and being selected from a group -(CH₂)ₙ-Y or -CH=CH-Y wherein Y represents a halogen atom, a radical -OH, -OR, -COH, -COR, -COOH, -COOR, -CONH₂, -CON (Rx,Ry), -CH=NOH, -CO-CH=NOH, -NH₂, -N(Rx, Ry), -NO₂, -PO(OR)₂, -SH₂, -SR, -SO₂R, -SO₂NHR, -CN or Z(R_{c}),
where R represents an alkyl radical of 1 to 8 carbon atoms,
Rₓ and R_{y}, which are the same or different, represent an alkyl radical of 1 to 5 carbon atoms,
- Z represents a phenyl radical and n is zero or an integer from 1 to 5, it being additionally possible for Rb to represent a hydrogen atom, and, when Y represents a group -COOH or -COOR in Rc, Z has at least 3 substituents or the quinoline ring system is tri-substituted and, when Y represents a group -CHO, Rc represents a group -(CH₂)ₙ-Y wherein n=1 to 5,
X represents an ethylenic double bond or a group -(CH₂)ₙ- where n is an integer from 1 to 5 or a group -CH(Rd) -CH(Re)-, Rd and Re, which are the same or different, representing a hydrogen or halogen atom, a hydroxy or epoxy group or a group -(CH₂)_{n'}-O-C(O)-(CH₂)ₘ-, -(CH₂)_{n'}-C(O)-(CH₂)ₘ-, -(CH₂)_{n'}-O-(CH₂)ₘ-, -(CH₂)n'-N(Q)-(CH2)ₘ- or -(CH₂)_{n'}-S(O)ₜ-(CH₂)ₘ-, where n' is an integer from 0 to 8, m is an integer from 0 to 8, t is zero or an integer equal to 1 or 2, and Q represents a hydrogen atom or an alkyl or phenyl radical, and also pharmaceutically acceptable salts of those derivatives, their diastereoisomeric forms and their enantiomeric forms, as an antiviral medicament.

11. Compound according to claim 10 for inhibiting HIV integrase.

12. Compound according to claim 10 for preventing or treating HIV.

13. Compound according to any one of claims 10 to 12 as defined according to any one of claims 2 to 4.

14. Use of a compound according to any one of claims 10 or 13 in the preparation of a medicament for preventing or treating HIV.

15. Combination comprising a compound according to claim 10 or 13 with another anti-HIV medicament.

16. Combination according to claim 14, wherein said anti-HIV medicament is a reverse transcriptase inhibitor and/or protease inhibitor.

## Patentansprüche

1. Chinolin-Derivate, **dadurch gekennzeichnet, dass** sie einer allgemeinen Formel II entsprechen: 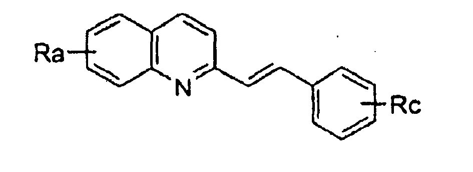 in welcher:
- Ra zwei Substituenten darstellt, wovon ein Substituent eine Gruppe -OH ist und der andere Substituent ausgewählt ist aus der Gruppe -(CH₂)ₙ-Y oder -CH=CH-Y, worin Y ein Halogenatom, einen Rest -OH, -OR, -COH, -COR, -COOH, - COOR, -CONH₂, -CON (Rx,Ry), -CH=NOH, -CO-CH=NOH, - NH₂, -N(Rx,Ry), -NO₂, -PO(OR)₂, -SH₂, -SR, -SO₂R, -SO₂NHR, -CN oder Z(R_{c}) entspricht,
worin R einem Alkylrest mit 1 bis 8 Kohlenstoffatomen entspricht, Rₓ und R_{y} gleich oder verschieden einen Alkylrest mit 1 bis 5 Kohlenstoffatomen darstellen und n gleich Null oder eine ganzzahlige Zahl von 1 bis 5 ist, Z einem Phenylrest entspricht;
- Rc zwei oder drei Substituenten -OH entspricht, sowie die pharmazeutisch annehmbaren Salze dieser Derivate, ihre diastereomeren Formen und ihre enantiomeren Formen.

2. Derivate nach Anspruch 1, in welchen Rₐ eine Gruppe darstellt, welche ausgewählt ist aus -CH=CH-COOH, -CH=CH-COOR, -CH=CH-COH, -CH=CH-COR, -CH=CH-CONH₂, -CON(Rₓ,R_{y}) und - CH=CH-Z(R_{c}).

3. Derivate nach den Ansprüchen 1 oder 2, **dadurch gekennzeichnet, dass** sie der Formel II entsprechen 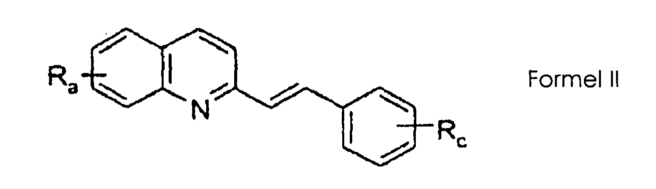 in welcher
- Rₐ zwei Substituenten darstellt, wovon einer eine Gruppe -OH ist und der andere ausgewählt ist aus OH und COOH und
- Rc zwei oder drei Substituenten -OH entspricht.

4. Chinolin-Derivate, **dadurch gekennzeichnet, dass** es sich um 8-Hydroxy-2-[2-[(3,4-dihydroxyphenyl)ethenyl]]-7-chinolincarbonsäure, dem Natriumsalz der 8-Hydroxy-2-[2-[(3,4-dihydroxyphenyl)ethenyl]]-7-chinolincarbonsäure, 7-Cyano-8-hydroxy-2-[2-(3,4-dihydroxyphenyl)-ethenyl]]-chinolin oder 8-Hydroxy-2-[2-[(3,4,5-trihydroxyphenyl)ethenyl]]-7-chinolincarbonsäure handelt.

5. Verfahren zur Synthese von Derivaten gemäß Anspruch 1, **dadurch gekennzeichnet, dass** es umfasst
- die Reaktion eines Chinolins der Formel III mit einem aromatischen Derivat oder einem heteroaromatischen Derivat der Formel IV, welches geeignete Blockierungsgruppen trägt: 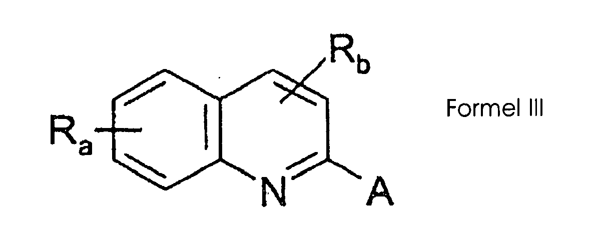
B-Z(R_{c}) Formel IV
in welchen A und B reaktive Gruppen darstellen, welche die Gruppe X ergeben, wie oben stehend definiert, Rₐ, R_{c} die gegebenen Bedeutungen im Verhältnis zur Formel II darstellen, jedoch Blockierungsgruppen umfassen und Rb = Wasserstoff und Z = Phenyl ist; und
- Entfernen der Schutzgruppen.

6. Verfahren nach Anspruch 5, **dadurch gekennzeichnet, dass** zur Herstellung der Derivate der Formel II
- man ein Chinolin der Formel V und ein aromatisches Derivat oder heteroaromatisches Derivat der Formel VI, welches geeignete Blockierungsgruppen umfasst, miteinander reagieren lässt, 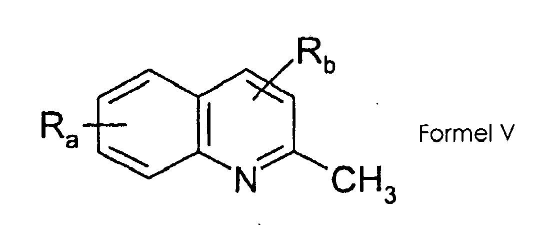
OHC-Z(R_{c}) Formel VI
in welchen die unterschiedlichen Substituenten die angegebenen Bedeutungen im Verhältnis zur Formel II aufweisen, jedoch Blockierungsgruppen tragen und Rb = Wasserstoff und Z = Phenyl ist;
- man diese Blockierungsgruppen eliminiert.

7. Verfahren nach Anspruch 6, **dadurch gekennzeichnet, dass** man zur Herstellung der Derivate der Formel II gemäß Anspruch 3 ein Chinolin der Formel V reagieren lässt, in welchem Rₐ mindestens einen Substituenten darstellt, welcher ausgewählt ist aus der Gruppe
-OH, -COOH oder einem Oxim und vorzugsweise zwei Substituenten, wovon ein Substituent eine Gruppe -OH ist und der andere Substituent eine der Bedeutungen aufweist, welche oben angegeben sind, mit einem Acetoxybenzaldehyd der Formel VII 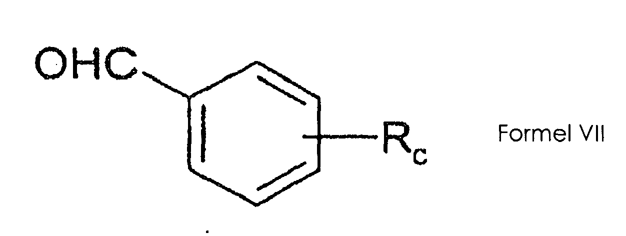 in welcher R_{c} mindestens zwei Gruppen -OH entspricht, welche durch Schutzgruppen blockiert sind.

8. Pharmazeutische Zusammensetzungen, **dadurch gekennzeichnet, dass** sie zusammen mit pharmazeutisch annehmbaren Trägern eine wirkungsvolle Menge mindestens eines Chinolin-Derivates der Formel (II) gemäß einem der Ansprüche 1 bis 4 umfasst.

9. Verwendung einer Verbindung der Formel (II) gemäß einem der Ansprüche 1 bis 4 als biologisches Reagenz.

10. Verbindung der Formel (I): 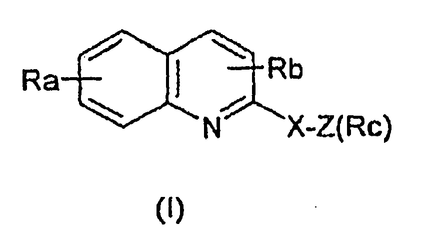 in welcher
- Ra, Rb und Rc, gleich oder verschieden, einen oder mehrere Substituenten darstellen, welche gleich oder verschieden sein können, und welche an eine beliebige Position an dem Ring gebunden sein können und welche ausgewählt sind aus einer Gruppe -(CH₂)ₙ-Y oder -CH=CH-Y, worin Y ein Halogenatom, ein Rest -OH, -OR, -COH, -COR, -COOH, -COOR, -CONH₂, -CON (Rx,Ry), -CH=NOH, -CO-CH=NOH, -NH₂, -N(Rx,Ry), -NO₂, - PO(OR)₂, -SH₂, -SR, -SO₂R, -SO₂NHR, -CN oder Z(R_{c}) entspricht,
worin R einem Alkylrest mit 1 bis 8 Kohlenstoffatomen entspricht, Rₓ und R_{y}, gleich oder verschieden, einem Alkylrest mit 1 bis 5 Kohlenstoffatomen entsprechen,
- Z einem Phenylrest entspricht und n gleich Null oder eine ganzzahlige Zahl von 1 bis 5 ist,
Rb unter anderem einem Wasserstoffatom entsprechen kann, und wenn Y eine Gruppe -COOH oder -COOR in Rc darstellt, Z mindestens 3 Substituenten trägt oder der Chinolin-Ring dreifach substituiert ist und wenn Y eine Gruppe -CHO entspricht, Rc eine Gruppe -(CH₂)ₙ-Y entspricht mit n=1 bis 5,
X eine ethylenische Doppelbindung oder einer Gruppe -(CH₂)ₙ-entspricht, in welcher n eine ganzzahlige Zahl von 1 bis 5 ist oder einer Gruppe -CH(Rd)-CH(Re)- entspricht, Rd und Re, gleich oder verschieden, ein Wasserstoffatom, ein Halogen, eine Hydroxygruppe oder eine Epoxygruppe oder einer Gruppe -(CH₂)ₙ,-O-C(O)-(CH₂)ₘ-,
- (CH₂)ₙ,-C(O)-(CH₂)ₘ-,-(CH₂)n,-O-(CH₂)ₘ-, -(CH₂)ₙ, -N(Q)-(CH₂)ₘ- oder
- (CH₂)ₙ,-S(O)ₜ-(CH₂)ₘ- entsprechen, worin n' eine ganzzahlige Zahl von 0 bis 8 ist, m eine ganzzahlige Zahl von 0 bis 8 ist, t gleich 0 oder eine ganzzahlige Zahl von 1 oder 2 ist und Q ein Wasserstoffatom, einem Alkylrest oder einem Phenylrest entspricht, sowie die pharmazeutisch annehmbaren Salze dieser Derivate, ihre diastereomeren Formen und ihre enantiomeren Formen als antivirales Medikament.

11. Verbindung nach Anspruch 10 zum Inhibieren der Infektion von HIV.

12. Verbindung nach Anspruch 10 zum Schützen gegen oder Behandeln von HIV.

13. Verbindung gemäß einem der Ansprüche 10 bis 12 wie definiert in einem der Ansprüche 2 bis 4.

14. Verwendung einer Zusammensetzung gemäß einem der Ansprüche 10 bis 13 für die Herstellung eines Medikamentes zum Schutz gegen oder des Behandelns von HIV.

15. Kombinationen, umfassend eine Verbindung gemäß dem Anspruch 10 oder 13 mit einem anderen Medikament gegen HIV.

16. Kombination nach Anspruch 14, worin das Medikament gegen HIV ein Inhibitor der reversen Transkriptase und/oder Protease ist.
